# DEMANDE DE BREVET EUROPEEN

(11) **EP 1 726 291 A1**
(43) Date de publication de la demande: **29.11.2006**
(21) Numéro de dépôt: 06113078.7
(22) Date de dépôt: 25.04.2006
(51) Int. Cl.: A61K 8/19, A61K 8/26, A61K 8/29, A61K 8/04, A61Q 1/14

(54) **Composition cosmétique à rincer contenant des particules interférentielles**

(30) Priorité: 25.05.2005 FR 0551371
(71) Demandeur: Societe L'Oreal S.A., 75008 Paris (FR)
(72) Inventeur: Sebillotte-Arnaud, Laurence, 94240, L'Hay les Roses (FR)
(74) Mandataire: Rasson, Catherine

(57) **Abrégé**

La présente demande concerne une composition cosmétique à rincer, sous forme d'une émulsion contenant une phase huileuse et une phase aqueuse, caractérisée en ce qu'elle contient des particules interférentielles hydrophiles choisies parmi les nacres non colorées recouvertes d'une ou plusieurs couches d'un ou plusieurs oxydes métalliques, ces nacres ayant une longueur maximale inférieure ou égale à 130 µm, et l'épaisseur d'oxydes métalliques étant supérieure à 80 nm, la quantité de phase interne étant supérieure à 50 % en poids par rapport au poids total de la composition.

La composition selon l'invention change de couleur au cours de l'application sur la peau et permet ainsi d'apprécier l'efficacité du démaquillage ou du soin de la peau.

## Description

L'invention a pour objet une composition cosmétique constituant des produits à rincer et contenant des particules interférentielles comme indicateurs d'efficacité, et à son utilisation comme produit de nettoyage ou de démaquillage de la peau du visage et/ou du corps, dont le cuir chevelu, des cheveux, ou comme produit de soin de la peau.

Le nettoyage de la peau est très important pour le soin du visage, et il doit être le plus performant possible car les résidus gras tels que l'excès de sébum, les restes des produits cosmétiques utilisés quotidiennement et les produits de maquillage notamment les produits « waterproof » résistants à l'eau, s'accumulent dans les replis cutanés et peuvent obstruer les pores de la peau et entraîner l'apparition de boutons.

Pour obtenir un bon démaquillage de la peau, il est connu d'utiliser des émulsions démaquillantes sous forme de laits ou de crèmes. Toutefois, il n'est pas toujours aisé de savoir quelle quantité de produit doit être appliquée sur le visage ni combien de temps il faut masser pour que le démaquillage soit optimum. Or, il est important que la peau soit complètement démaquillée pour les raisons indiquées ci-dessus.

Il subsiste donc le besoin d'un produit qui, tout en démaquillant ou nettoyant, indique l'efficacité du démaquillage ou nettoyage, et qui permette à l'utilisatrice de produits de maquillage de savoir si elle est suffisamment démaquillée ou non.

La demanderesse a découvert de manière surprenante qu'on pouvait atteindre le but de l'invention par addition de particules interférentielles hydrophiles dans les produits démaquillants. Ces pigments changent de couleur au fur et à mesure de l'application, indiquant ainsi par ce changement de couleur, l'efficacité du démaquillage. Par ailleurs, l'addition de ces pigments rend plus ludique l'action de démaquillage. Cet effet de changement de couleur peut être aussi utilisé pour indiquer l'efficacité des produits de soin rincés.

Certes, il a été décrit des compositions cosmétiques rincées contenant des pigments brillants, notamment dans les documents US-A-6,759,376 et WO-A-2004/100921. Toutefois, il s'agit généralement de pigments rendus hydrophobes, et en plus, le but en est le dépôt de ces pigments sur la peau, la modification hydrophobe des particules et la présence d'émollient favorisant la rémanence de ces pigments après rinçage de ces pigments, ce qui conduit à un dépôt brillant sur la peau. En outre, ces pigments ne donnent pas d'effet de changement de couleur au cours de l'application sur la peau.

Au contraire, les pigments utilisés dans la présente invention, du fait de leur propriété hydrophile, ne laissent pas de dépôt brillant à la surface de la peau après rinçage, dépôt qui serait alors synonyme d'une peau mal démaquillée ou mal nettoyée dans le cadre de la présente invention.

La présente demande a donc pour objet une composition cosmétique à rincer, sous forme d'une émulsion contenant une phase huileuse et une phase aqueuse, caractérisée en ce qu'elle contient des particules interférentielles hydrophiles choisies parmi les nacres non colorées recouvertes d'une ou plusieurs couches d'un ou plusieurs oxydes métalliques, ces nacres ayant une longueur maximale inférieure ou égale à 130 µm, l'épaisseur d'oxydes métalliques étant supérieure à 80 nm, la quantité de phase interne étant supérieure à 50 % en poids par rapport au poids total de la composition.

La composition étant destinée à une application topique, elle comprend un milieu physiologiquement acceptable. Par ailleurs, on entend par « milieu physiologiquement acceptable », un milieu compatible avec la peau, les lèvres, le cuir chevelu, les cils, les yeux, les ongles et/ou les cheveux.

La composition selon l'invention présente l'avantage d'être facile à appliquer, de permettre un démaquillage rapide et efficace, d'être facile à éliminer sans laisser la peau grasse ou brillante et de laisser la peau confortable après utilisation (pas de tiraillement ni de sécheresse).

Les compositions de l'invention sont des compositions à rincer (rinçage à l'eau ou avec un tonique), et elles sont utilisables dans le domaine du démaquillage du nettoyage de la peau du visage ou du corps, des cheveux dont le cuir chevelu, et des muqueuses telles que les lèvres. Elles peuvent constituer également des produits de soin comme par exemple des masques à rincer (de la manière habituelle d'utilisation de ces produits), des produits désincrustants ou exfoliants aussi bien pour le visage que pour les mains (lorsque la composition contient des particules exfoliantes).

L'utilisation comme désincrustant ou exfoliant consiste généralement à appliquer le produit sur le visage ou les mains ou le corps, à frotter une minute ou deux puis à rincer. La peau est alors lisse, douce et désincrustée,

Les utilisations indiquées ci-dessus sont faites de la manière habituelle d'utilisation de ces produits.

La présente invention a encore pour objet l'utilisation cosmétique de la composition telle que définie ci-dessus, pour le démaquillage et/ou le nettoyage de la peau, des cheveux et/ou des muqueuse, ou le soin de la peau.

D'une manière générale, la composition conforme à l'invention est blanche dans la masse, c'est-à-dire qu'elle est blanche dans le récipient la contenant, à condition qu'elle ne contienne pas, en plus des particules interférentielles, des colorants ou des pigments colorants classiques qui pourraient colorer la masse. L'ajout de tels colorants ou pigments colorants est possible pour modifier la couleur de la composition mais cet ajout ne doit pas interférer sur l'effet de changement de couleur à l'application sur la peau. De manière préférée, la composition selon l'invention est exempte de pigments ayant un effet filtrant, et de manière générale, elle est exempte de filtres UV inorganique et organique.

Lorsqu'elle ne contient ni colorant ni pigment colorant, l'aspect de la composition telle qu'elle se présente dans la masse doit être incolore (blanc) ou légèrement nacré. Ainsi, la composition selon l'invention est, en outre, caractérisée par des valeurs colorimétriques de clarté L* et de saturation C* mesurées dans l'espace colorimétrique CIE 1976, ces valeurs étant mesurées en absence de colorant et/ou de pigment colorant dans la composition.

Les mesures colorimétriques L* et C* peuvent être réalisées à l'aide d'un colorimétrique CHROMAMETER CR400® de MINOLTA. Comme indiqué ci-dessus, ces mesures colorimétriques doivent être réalisées sur la composition contenant les particules interférentielles mais avant ajout de tout colorant ou pigment colorant.

Pour faire les mesures, la composition à tester est introduite dans un pot de volume de 15 ml (diamètre d'ouverture : 1,9 cm ; profondeur : 1,8 cm). La surface de la composition introduite est lissée par arasement avec une lame de verre. La cellule du colorimètre est alors mise en contact avec cette surface et les paramètres colorimétriques sont déterminés. En absence de colorant et/ou de pigment colorant, les compositions selon l'invention se caractérisent par une clarté L* supérieure à 60, de préférence supérieure à 75 et plus préférentiellement supérieure à 80, associée à une saturation C* inférieure à 10, de préférence inférieure à 5 et plus préférentiellement inférieure à 3, ce qui montre que la composition est blanche.

Pour information, la référence blanche fournie avec le colorimètre CHROMAMETER CR400® est caractérisée par les valeurs de clarté L* et de saturation C* suivantes : L* = 96,94+/- 0,01, C* = 2,83+/- 0,01.

Les compositions de l'invention sont caractérisées par des changements de couleur entre la couleur en pot, la couleur à l'application sur la peau, et la couleur après massage sur la peau. Ces changements de couleur sont fonction du sens de l'émulsion, du cisaillement de la composition sur la peau, de l'épaisseur de la couche déposée sur la peau, de l'état physique du produit sur la peau, par exemple si le produit est en deux phases après dépôt sur la peau (eau d'un coté, huile de l'autre), ou si le produit est resté sous forme d'émulsion sur la peau.

Pour utiliser la composition de l'invention, on la met sur la peau en une couche épaisse, par exemple une couche de 15 à 35 mg/cm², et au moment du massage sur la peau, on l'étale pour avoir une couche mince. Les phénomènes produits sont différents selon que la composition est sous forme d'émulsion huile-dans-eau (H/E) (émulsion directe avec phase huileuse dispersée dans la phase aqueuse) ou sous forme d'émulsion eau-dans-huile (E/H) (émulsion inverse avec phase aqueuse dispersée dans la phase huileuse).

Lors du massage, les émulsions H/E cassent sur la peau en libérant l'huile alors que les émulsions E/H cassent en libérant de l'eau. Ainsi, il y a deux évolutions possibles selon le sens de l'émulsion : dans le cas des émulsions H/E, il y a une couleur lorsque le produit est en couche épaisse puis disparition de la couleur. L'utilisateur sait que l'épaisseur de la couche appliquée et donc la quantité de composition appliquée sur la peau sont suffisantes si une couleur apparaît à l'oeil. La couche épaisse est appliquée sur toute la zone à traiter, par exemple à démaquiller, par exemple sur tout le visage. Après application, on masse, et la disparition de la couleur se fait en même temps que l'huile est libérée, et ainsi quand la couleur n'est plus perceptible par l'utilisateur, cela signifie que le massage peut être arrêté et que l'huile libérée va permettre le démaquillage ou le soin.

Dans le cas d'une émulsion E/H, il n'y a pas de couleur lors de l'application sur la peau en couche épaisse, mais la couleur apparaît lorsque l'émulsion casse en libérant l'eau et les particules interférentielles à la surface de la peau, ce qui signifie que le massage peut être arrêté.

Ensuite, dans les deux cas, on rince de telle sorte qu'il ne reste plus de particules brillantes sur la peau, les particules interférentielles ayant eu un rôle d'indicateur de démaquillage, de nettoyage ou de soin, mais ne restant pas sur la peau après rinçage.

L'invention a aussi pour objet un procédé de démaquillage et/ou de nettoyage de la peau et/ou des cheveux et/ou des muqueuses, ou de soin de la peau, consistant à :
1) appliquer sur la zone à démaquiller ou à nettoyer ou à soigner, la composition selon l'invention en une épaisseur suffisante, par exemple une couche d'une épaisseur de 15 à 35 mg/cm²,
2) masser la composition sur la zone pour l'étaler jusqu'à disparition de la couleur si la composition est une émulsion H/E ou jusqu'à apparition de la couleur si la composition est une émulsion E/H,
3) rincer jusqu'à élimination de la composition.

### Particules interférentielles

Au sens de la présente invention, l'expression "particule interférentielle" désigne toute particule possédant généralement une structure multicouche telle qu'elle permette la création d'un effet de couleur par interférence des rayons lumineux qui diffractent et diffusent différemment selon la nature des couches. Ainsi, ces particules peuvent présenter des couleurs variant selon l'angle d'observation et l'incidence de la lumière.

Au sens de la présente invention, une structure multicouche entend désigner indifféremment une structure formée d'un substrat recouvert d'une unique couche ou une structure formée d'un substrat recouvert d'au moins deux voire de plusieurs couches consécutives.

Les particules interférentielles utilisées selon l'invention sont des nacres, c'est-à-dire des particules constituées de mica. Ces micas sont recouverts d'oxyde métallique. La couleur de la particule dépend de l'épaisseur de la couche d'oxyde métallique. De préférence, les nacres sont des micas recouverts d'oxyde de titane (TiO₂) comme oxyde métallique.

| Couleur du pigment | Epaisseur de TiO₂ |
|---|---|
| Argent | 40 à 60 nm |
| Or | 60 à 80 nm |
| Rouge | 80 à 100 nm |
| Bleu | 100 à 140 nm |
| Vert | 120 à 160 nm |

L'épaisseur de la couche d'oxyde métallique et notamment d'oxyde de titane des nacres utilisées selon l'invention doit être supérieure à 80 nm, ce qui permet la formation d'une couleur différente de l'argent ou de l'or. Les particules interférentielles peuvent comprendre en outre une couche d'un autre oxyde métallique tel que l'oxyde d'étain et/ou la silice (ou oxyde de silicium, SiO₂), afin de moduler la couleur obtenue par le mica recouvert d'une couche d'oxyde de titane. Ainsi par exemple, la couleur peut être modifiée en fonction de l'épaisseur de la couche d'oxyde d'étain, on peut aussi ajouter une couche de silice (SiO₂) pour augmenter la quantité de lumière réfléchie.

D'autre part, la longueur maximale de la nacre utilisée selon l'invention ne doit pas dépasser 130 µm, ceci pour permettre l'obtention d'une couleur homogène à l'étalement (les particules sont visibles si la taille est supérieure). On entend par « longueur maximale », la plus grande longueur des particules, étant entendu que les particules commercialement disponibles sont un mélange de particules ayant différentes longueurs, comme on le voit dans la liste de matières premières indiquées plus loin. Dans la composition selon l'invention, la longueur maximale de particules ne doit pas dépasser 130 µm. L'exemple comparatif 6 présenté ci-après montre que, lorsque la longueur maximale dépasse 130 µm, la composition obtenue ne donne pas l'effet recherché. De préférence, la longueur de la nacre va de 5 à 130 µm et mieux de 5 à 125 µm.

Comme particules interférentielles particulièrement appropriées pour la composition de l'invention, on peut citer les nacres de type :
- mica / oxyde de titane / oxyde d'étain, telles que par exemple celles commercialisées sous les dénominations TIMIRON SILK BLUE^{®}, TIMIRON SILK RED^{®}, TIMIRON SILK GREEN^{®}, TIMIRON SILK VIOLET^{®} par la société MERCK, celle commercialisée sous la dénomination XIRONA^{®} Volcanic fire par la société MERCK, ou celles de la gamme Total PRESTIGE^{®}, Silk orange, Silk Red, Silk Violet, Silk Blue, Silk Green commercialisées par la Société ECKART ;
- mica / oxyde de titane, telles que par exemple celles commercialisées sous les dénominations FLAMENCO BLUE^{®}, FLAMENCO RED^{®} et FLAMENCO GREEN^{®} par la société ENGELHARD,
- et leurs mélanges.

Ces particules interférentielles peuvent être présentes dans la composition de l'invention en une quantité allant par exemple de 0,5 à 5 % en poids et de préférence de 1 à 4 % en poids par rapport au poids total de la composition.

### Emulsion

L'émulsion constituant le support de la composition comprend une phase aqueuse et une phase huileuse, et selon le sens de l'émulsion, la composition peut être sous forme d'émulsion H/E ou d'émulsion E/H.

L'émulsion peut être préparée par tout procédé d'émulsification classique. Dans le cas des émulsions H/E, elle peut être obtenue aussi par la technique d'inversion de phase en température.

Les émulsions selon l'invention ont une quantité de phase interne supérieure à la quantité de phase externe. Ainsi, elles contiennent une quantité de phase interne supérieure à 50 % en poids par rapport au poids total de la composition (phase huileuse pour les H/E et phase aqueuse pour les E/H) et de préférence plus de 60% et plus préférentiellement de 60 à 95% et encore préférentiellement de 60% à 90% en poids de phase interne dispersée par rapport au poids total de la composition. L'émulsion ainsi obtenue casse facilement à la surface de la peau au cours du massage. Cette modification sur la peau induit des variations de couleurs plus visibles par rapport à des émulsions qui ne cassent pas à la surface de la peau.

La phase huileuse contient au moins une huile, notamment une huile cosmétique. On entend par "huile" un corps gras liquide à la température ambiante (25°C). Comme indiqué ci-dessus, la quantité relative des deux phases dépend du sens de l'émulsion. Pour les émulsions H/E, la quantité de phase huileuse peut aller par exemple de plus de 50 % à 95% en poids, de préférence de 55 à 95 % en poids et mieux de 60 à 90% en poids par rapport au poids total de la composition. Pour les émulsions E/H, la quantité de phase huileuse peut aller par exemple de 5 à moins de 50 %, de préférence de 5 à 45 % en poids et mieux de 10 à 40% en poids par rapport au poids total de la composition.

Pour les phases aqueuses, les quantités sont inversées, c'est-à-dire que pour les émulsions H/E, la quantité de phase aqueuse peut aller par exemple de 5 à moins de 50 %, de préférence de 5 à 45 % en poids et mieux de 10 à 40 % en poids par rapport au poids total de la composition. Pour les émulsions E/H, la quantité de phase aqueuse peut aller par exemple de plus de 50 % à 95% en poids, de préférence de 55 à 95 % en poids et mieux de 60 à 90% en poids par rapport au poids total de la composition.

### Phase huileuse

La phase huileuse de la composition selon l'invention contient tous les corps gras et additifs lipophiles. Elle contient au moins une huile, notamment une huile cosmétique. On entend par "huile" un corps gras liquide à la température ambiante (25°C).

Selon un mode préférée de réalisation de l'invention, la phase huileuse contient au moins une huile choisie parmi les huiles hydrocarbonées, les esters d'acide gras (ou esters gras), les éthers d'alcool gras (ou éthers gras), et leurs mélanges. On entend par «huile hydrocarbonée», toute huile comportant majoritairement des atomes de carbone et d'hydrogène, et éventuellement des groupements ester, éther, fluoré, acide carboxylique et/ou alcool.
- les huiles hydrocarbonées linéaires ou ramifiés, d'origine minérale, synthétique ou animale, sont choisies parmi les huiles de paraffine (Mineral oil), et leurs dérivés, la vaseline, l'huile de vaseline, le perhydrosqualène, les polydécènes, l'isohexadecane, l'isododecane, le polyisobutène hydrogéné (ou isoporaffine hydrogénée) tel que l'huile de Parléam.
- Les esters gras sont de préférence ceux obtenus à partir d'un alcool à chaîne linéaire ou ramifiée, ayant de 1 à 17 atomes de carbone et d'un acide gras à chaîne linéaire ou ramifiée, ayant de 3 à 18 et de préférence de 12 à 17 atomes de carbone. De façon avantageuse, ledit ester est un ester saturé qui ne renferme aucun groupement éther ni hydroxyle (la quantité totale de carbone dans l'ester peut varier de 12 à 50 et de préférence de 20 à 50).

Comme esters gras, on peut citer par exemple le caprate/caprylate d'éthyl-2 hexyle (ou caprate/caprylate d'octyle), le laurate d'éthyle, le laurate de butyle, le laurate d'hexyle, le laurate d'isohexyle, le laurate d'isopropyle, le myristate de méthyle, le myristate d'éthyle, le myristate de butyle, le myristate d'isobutyle, le myristate d'isopropyle, le myristate d'octyl-2 dodécyle, le monococoate d'éthyl-2 hexyle (ou monococoate d'octyle) , le palmitate de méthyle, le palmitate d'éthyle, le palmitate d'isopropyle, le palmitate d'isobutyle, le palmitate d'ethyl-2 hexyle (ou palmitate d'octyle), le stéarate de butyle, le stéarate d'isopropyle, le stéarate d'isobutyle, le stéarate d'isocétyle, l'isostéarate d'isotéaryle, l'isostéarate d'isopropyle, le stéarate d'éthyl-2 hexyle (ou stéarate d'octyle), le pelargonate d'ethyl-2 hexyle (ou pelargonate d'octyle), l'hydroxystéarate d'éthyl-2 hexyle (ou hydroxystéarate d'octyle), le decyl oleate, l'adipate de di-isopropyle, l'adipate de di-éthyl-2 hexyle (ou adipate de di-octyle), l'adipate de diisocetyle, le succinate d'ethyl-2 hexyle (ou succinate d'octyle), le sébacate de diisopropyle, le malate d'ethyl-2 hexyle (ou malate d'octyle), le caprate/caprylate de pentaérythritol, le pentaérythritol de tétraisostéarate, l'hexanoate d'éthyl-2 hexyle (ou hexanoate d'octyle), l'octanoate d'octyldodécyle, le néopentanoate d'isodécyle, le néopentanoate d'isostéaryle, l'isononanoate de cétéaryle, l'isononanoate d'isodécyle, l'isononanoate d'isononyle, l'isononanoate d'isotridécyle, le lactate de lauryle, le lactate de myristyle, le lactate de cétyle, le propionate de myristyle, l'éthyl-2 hexanoate d'éthyl-2 hexyle (ou éthyl-2 hexanoate d'octyle), l'octanoate d'éthyl-2 hexyle (ou octanoate d'octyle, et leurs mélanges, benzoates d'alcools gras en C12-C15 (Finsolv TN de FINETEX) le lauroyl sarcosinate d'isopropyle (Eldew SL 205 de Unipex), le dicaprylyl carbonate (Cetiol CC de la société Cognis).
- Comme éthers gras, on peut citer le Dicaprylyl ether (Cetiol OE de la société Cognis).

La composition peut contenir en outre une ou plusieurs autres huiles choisies parmi les huiles végétales, les huiles de silicone, les huiles fluorées, volatiles ou non. On peut notamment citer :
- les huiles hydrocarbonées d'origine végétale telles que l'huile d'amande douce, l'huile d'avocat, l'huile de ricin, l'huile de coriandre, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore, l'huile de seigle, les triglycérides des acides caprylique/caprique comme ceux vendus par la société Stearineries Dubois ou ceux vendus sous les dénominations Miglyol 810, 812 et 818 par la société Dynamit Nobel ;
- les huiles de silicone, volatiles ou non, comme les polydiméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, les polyméthyl-phénylsiloxanes ;
- les huiles fluorées telles que celles partiellement hydrocarbonées et/ou siliconées comme celles décrites dans le document JP-A-2-295912.

La phase huileuse de la composition selon l'invention peut également comprendre d'autres corps gras que les huiles, et notamment un ou plusieurs composés choisis parmi les cires, les gommes, les corps gras pâteux, ces composés pouvant être d'origine végétale, animale, minérale ou de synthèse, siliconé ou non. Les cires peuvent être hydrocarbonées, siliconées et/ou fluorées, comportant éventuellement des fonctions ester ou hydroxyle. La cire ou les cires peuvent représenter de 0 à 10 % en poids, notamment de 0,05 à 5 % en poids, par rapport au poids total de la composition.

### Phase aqueuse

La phase aqueuse des compositions selon l'invention peut contenir, outre l'eau, un ou plusieurs solvants choisis parmi les mono-alcools comportant de 1 à 6 atomes de carbone, les polyols et leurs mélanges. Comme mono-alcools, on peut citer notamment l'éthanol. Comme polyols, on peut citer notamment la glycérine ; les glycols comme le butylène glycol, l'isoprène glycol, le propylène glycol, les polyéthylène glycols tels que le PEG-8 ; le sorbitol ; les sucres tels que le glucose, le fructose, le maltose, le lactose, le sucrose ; et leurs mélanges. Lorsqu'ils sont présents, la quantité de mono-alcools et de polyols dans la composition de l'invention peut aller par exemple de 0,01 à 30 % en poids, de préférence de 2 à 25 % en poids et mieux de 5 à 20 % en poids par rapport au poids total de la composition.

### Emulsionnants

La composition selon l'invention constitue une émulsion, et la dispersion d'une phase dans l'autre est de préférence réalisée en présence d'au moins un émulsionnant qui est différent selon le sens de l'émulsion. La quantité en émulsionnant peut aller par exemple de 1 à 20 % en poids, de préférence de 1 à 15 % en poids et mieux de 2 à 10 % en poids par rapport au poids total de la composition.

Selon le sens de l'émulsion, l'émulsionnant peut être introduit dans la phase aqueuse ou dans la phase huileuse.

Les émulsions H/E comprennent de préférence un émulsionnant ou un mélange d'émulsionnants, ayant un HLB (Hydrophilic Lipophilic balance) allant de 8 à 18. Par ailleurs, la solubilité de certains émulsionnants dans les huiles peut éventuellement augmenter avec la température, ce qui permet l'obtention d'émulsions par le procédé d'inversion de phase en température.

Les émulsions E/H comprennent de préférence un émulsionnant ou un mélange d'émulsionnants, ayant un HLB (Hydrophilic Lipophilic balance) inférieur à 8.

Les émulsionnants des émulsions H/E peuvent être choisis parmi les alcools gras éthoxylés, les acides gras éthoxylés, les esters d'acide gras et de sucre ou de sorbitan, les glycérides partiels d'acides gras ethoxylés, les triglycérides d'acides gras polyglycérolés ethoxylés ou non et leurs mélanges, tout autre émulsionnant classique approprié.

Les émulsionnants préférés sont les alcools gras éthoxylés ou les acides gras éthoxylés de formules suivantes :

R-O-(CH₂-CH₂-O)ₘH

Ou bien

R-COO-(CH₂-CH₂-O)ₘH

où R est une chaîne hydrocarbonée linéaire ou ramifiée, saturée ou insaturée, possédant un nombre de carbones allant de 10 à 24 et m est compris entre 8 et 100.

Les alcools éthoxylés sont les éthers d'alcools gras comportant par exemple de 10 à 100 et de préférence de 10 à 50 groupes oxyéthylénés, tels que les Laureth-10 à 12, les Ceteth-10 à 30, les Steareth-10 à 30, les Ceteareth-10 à 30, les Isosteareth-10 à 50, les Beheneth-10 à 50.

Les acides éthoxylés sont les esters d'acide gras comportant par exemple de 10 à 150 et de préférence de 10 à 100 groupes oxyéthylénés, tels que les PEG-10 à PEG 50 laurate ou palmitate ou stearate ou palmito-stearate ou behenate. On peut citer plus particulièrement les stéarates de polyéthylène glycol comme le stéarate de PEG-100, le stéarate de PEG-50 et le stéarate de PEG-40 ; et les mélanges les contenant tels que le mélange de stéarate de glycéryle et de stéarate de PEG-100, commercialisé sous la dénomination Arlacel 165 par la société Uniqema et sous la dénomination SIMULSOL 165 par la société SEPPIC ; les esters d'acides gras et de sorbitan oxyéthylénés comprenant par exemple de 20 à 100 OE, et par exemple ceux commercialisés sous les dénominations commerciales Tween 20 ou Tween 60 par la société Ubiqema ; les esters de sucres oxyéthylénés, comme le PEG-20 méthylglucose sesquistéarate.

Comme esters d'acide gras et de sucre ou de sorbitan, on peut citer les esters de sucrose tels que le stéarate de sucrose, les esters de sorbitan tels que le palmitate de sorbitan commercialisé sous la dénomination Span 40 par la société Uniqema.

On peut citer aussi comme émulsionnant, tartrate de dimyristyle et les mélanges le contenant, tels que le mélange de tartrate de dimyristyle, d'alcool cétéarylique, de Pareth-7 et de PEG-25 laureth-25, commercialisé sous la dénomination Cosmacol PSE par la société Sasol (nom CTFA : Dimyristyl tartrate / cetearyl alcool /12-15 Pareth 7 / PPG 25 laureth 25).

D'autres émulsionnants peuvent être choisis parmi les mono- ou di-glycérides d'acides gras, d'acides gras polyglycérolés ou encore de triglycérides ethoxylés et d'autres émulsionnants classiquement utilisés tels que les alkylpolyglucosides, notamment ceux commercialisés sous les dénominations Montanov par la société Seppic.

On peut ajouter à ces émulsionnants, des co-émulsionnants tels que par exemple les alcools gras ayant de 12 à 30 atomes de carbone, comme l'alcool cétylique, l'alcool stéarylique et leur mélange (alcool cétéarylique), l'alcool béhénique et leurs mélanges.

Comme émulsionnants utilisables pour les émulsions E/H, on peut citer par exemple les esters d'acide gras et de polyéthylène glycol de HLB approprié, tels que le PEG-30 dipolyhydroxystearate commercialisé sous la dénomination ARLACEL P135 par la société Uniqema ; les dérivés glycérolés comme par exemple l'isostéarate de polyglycerol-4 ; les dérivés de Polyisobutenylsuccinate comme par exemple le mélange TEA-diéthanolaminoéthyl polyisobuténylsuccinate / ethylhexyl palmitate, commercialisé sous la dénomination CHEMCINNATE 2000 par la société Chemron ; les oligomères ou polymères dérivés de polyoléfine comme les produits commercialisés sous les dénominations LUBRIZOL 2724 et LUBRIZOL 5603 par la société Lubrizol. ; les tensioactifs siliconés comme les dimethicone copolyols tels que le mélange de cyclomethicone et de dimethicone copolyol, vendu sous les dénominations DC 5225 C et DC 3225 C par la société Dow Corning, et comme les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90^{R} par la société Goldschmidt et le mélange de Polyglyceryl-4 isostearate/Cetyl dimethicone copolyol/Hexyl laurate vendu sous la dénomination Abil WE 09^{R} par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan Gl 34 par la société Goldschmidt, l'isostéarate de sorbitan, tel que le produit commercialisé sous la dénomination Arlacel 987 par la société Uniqema, l'isostéarate de sorbitan et de glycérol, tel que le produit commercialisé sous la dénomination Arlacel 986 par la société Uniqema, et leurs mélanges.

### Additifs

La composition selon l'invention peut contenir différents additifs ou actifs, hydrosolubles ou liposolubles, choisis parmi ceux classiquement utilisés dans les produits de soin ou de démaquillage de la peau, dans la mesure où ces additifs et leurs quantités ne nuisent pas aux qualités recherchées pour la composition selon l'invention.

On peut citer en particulier comme adjuvants, les charges minérales ou organiques après avoir vérifié qu'elles ne nuisent pas à la variation de couleur des produits.

Les charges minérales et organiques, poreuses ou non, sphériques ou non peuvent être choisies parmi les oxydes minéraux ou les charges organiques.

Parmi les oxydes minéraux, on peut citer les oxydes de titane, de zinc, de fer, d'aluminium, de zirconium, de sélénium, de strontium, de manganèse, et plus préférentiellement ceux de silicium dont les silices. La silice peut être choisie parmi les silices hydrophiles, les silices hydrophobes et leurs mélanges. On entend par « silice » aussi bien les silices pures (hydrophiles ou hydrophobes) que les particules enrobées de silice. Ces silices sont de préférence amorphes et elles peuvent être d'origine pyrogénée ou d'origine précipitée. Elles peuvent se présenter sous forme pulvérulente ou en dispersion aqueuse. Elles sont caractérisées par une surface spécifique de 30 à 500 m2/g, une dimension moyenne de particules en nombre allant de 3 à 50 nm et une densité tassée allant de 40 à 200 g/l et mieux de 50 à 150 g/l. On peut citer par exemple comme silices, celles commercialisées sous les dénominations AEROSIL 90®, 130®, 150®, 200®, 300®, 380®, OX 50®, FK 320 DS®, R202®, R805®, R812®, R972®, R974® par la Société Degussa-Hüls.

D'autres charges minérales peuvent être utilisées. Ce sont par exemple les silicates d'aluminium (montmorillonnites, kaolinites), les silicates de magnésium (talc), les silicates d'aluminium et de magnésium (bentonites, argiles, attapulgites, smectites), les silicates doubles de calcium et de magnésium (les dolomies), les silicates d'aluminium et de sodium. Ainsi, on peut choisir par exemple le talc commercialisé sous la dénomination LUZENAC 15 M00® par la société Luzenac, le kaolin ou silicate d'aluminium commercialisé sous la dénomination KAOLIN SUPREME® par la société lmerys.

Les charges organiques peuvent être choisies par exemple parmi
- des microsphères de polymères synthétiques comme par exemple celles de Nylon (ORGASOL 2002 UD NAT COS® de la société Atochem), celles à base de copolymère de chlorure de vinylidene/Acrylonitrile/methacrylonitrile enfermant de l'isobutane, expansées (EXPANCEL 551 DE® par la société ExpanceL),
- des fibres de nylon 6,6 (POLYAMIDE 0.9 DTEX 0.3 MM des Etablissements Paul Bonte), de cellulose ou « Rayonne » (RAYON FLOCK RCISE N0003 MO4® de la société Claremont) ou encore d'amidon (AMIDON DE MAIS B ® de la société Roquette)

Comme additifs autres que les charges, on peut citer par exemple les adjuvants habituels dans le domaine cosmétique tels que les tensioactifs moussants ou détergents soit anioniques comme les lauryl ether sulfates de sodium, les alkyl phosphates de sodium, le trideceth sulfate de sodium, soit amphotères comme les alkyl bétaines, le disodium cocoamphodiacetate, soit non ioniques de HLB supérieure à 10 comme les POE/PPG/POE (nom CTFA: Poloxamer), les alkyl polyglucosides (ou APG), le polyglyceryl-3 hydroxylauryl ether ; les conservateurs ; les séquestrants (EDTA et ses sels) ; les antioxydants ; les parfums ; les matières colorantes ; les colorants solubles ou les pigments encapsulés ou non ; les filtres solaires ; des polymères hydrophiles ou lipophiles, anioniques, non ioniques, cationiques ou amphotères, épaississants ou dispersants ; les actifs cosmétiques ou dermatologiques hydrophiles ou lipophiles. Les quantités de ces différents adjuvants sont celles classiquement utilisées dans le domaine considéré, et par exemple de 0,01 à 20 % du poids total de la composition. Ces adjuvants ainsi que leurs quantités doivent être tels qu'ils ne modifient pas la propriété recherchée pour la composition de l'invention.

Comme agents actifs habituels dans le domaine cosmétique ou dermatologique pouvant être utilisés selon l'invention, on peut citer par exemple les vitamines, telles que par exemple les vitamines A, B3, PP, B5, E, K1 et/ou C et les dérivés de ces vitamines et notamment leurs esters ; les agents kératolytiques ou prodesquamants, par exemple les α-hydroxy-acides, les β-hydroxy-acides, les α-cétoacides, les β-ceto-acides, les rétinoïdes et leurs esters, le rétinal, l'acide rétinoïque et ses dérivés ; les extraits de plantes tels que les extraits de petit houx et/ou de marron d'Inde ; les bases xanthiques telles que la caféine ; les agents anti-radicaux libres ; les filtres solaires ; les agents hydratants comme les polyols ; les céramides ; la DHEA et ses dérivés ; le coenzyme Q10 ; les agents blanchissants et dépigmentants par action biologique comme l'acide kojique, les extraits de scutellaire, de mûrier, de réglisse et/ou de camomille 20 ; les dérivés de para-aminophénols, l'arbutine et leurs dérivés, et leurs mélanges.

Pour une utilisation dans le traitement cosmétique des peaux grasses ou mixtes, la composition selon l'invention contiendra en particulier au moins un actif choisi parmi les vitamines B3 et B5 ; les sels de zinc, et en particulier l'oxyde de zinc et le gluconate de zinc ; l'acide salicylique et ses dérivés tels que l'acide n-octanoyl-5-salicylique ; le triclosan ; la capryloylglycine ; un extrait de clou de girofle ; l'octopirox ; l'hexamidine ; et l'acide azélaïque et ses dérivés. En cas d'incompatibilité ou pour les stabiliser, les actifs mentionnés ci-dessus peuvent être incorporés dans des sphérules, notamment des vésicules ioniques ou non-ioniques et/ou des nanoparticules (nanocapsules et/ou nanosphères).

Les émulsions selon l'invention peuvent être préparées par tout procédé classique d'émulsification.

Selon un mode particulier de réalisation de l'invention, quand ce sont des émulsions H/E, celles-ci peuvent être obtenues par le procédé d'inversion de phase. Ce procédé de préparation consiste à :
- 1) Peser dans un récipient tous les constituants de la composition (à l'exception des matières premières thermosensibles et des charges)
- 2) Homogénéiser le mélange, par exemple au moyen d'un Rayneri 350 tours/min, et chauffer en augmentant progressivement la température au moyen d'un bain marie jusqu'à une température supérieure ou égale à la température d'inversion de phase T2, c'est-à-dire jusqu'à l'obtention d'une phase transparente ou translucide (zone de microémulsion ou de phase lamellaire) puis d'une phase blanche plus visqueuse qui indique l'obtention de l'émulsion inverse (E/H).
- 3) Arrêter le chauffage et maintenir l'agitation jusqu'à retour à la température ambiante, en passant par la température d'inversion de phase T1, c'est-à-dire la température à laquelle se forme une émulsion H/E fine.
- 4) Lorsque la température est redescendue en dessous de la zone d'inversion de Phase en Température (T1), additionner les charges et éventuellement les matières premières thermosensibles.

On obtient une émulsion H/E stable dont les gouttelettes d'huile sont très fines (taille moyenne en volume de globules d'huile allant de 50 nm à 1000 nm, de préférence, de 70 nm à 350 nm et plus particulièrement de 70 à 300 nm).

Les exemples indiqués ci-dessous permettront de mieux comprendre l'invention sans toutefois présenter un caractère limitatif. Les quantités indiquées sont en % en poids sauf mention contraire. Les noms sont en nom chimique, en nom commercial et en nom CTFA selon les composés.

### Exemples 1 à 9

Les exemples 1 à 5 sont des exemples selon l'invention tandis que les exemples 6 à 9 sont des exemples comparatifs. Tous ces exemples sont des émulsions H/E. Ces exemples ont été réalisés selon le procédé d'inversion de phase décrit ci-dessus.

Dans chaque exemple, une nacre différente a été introduite à une quantité de 1,5 %. Les compositions obtenues ont été étalées en une quantité de 15 mg/cm² sur une carte de contraste blanche et noire afin d'en déduire l'effet couleur qui sera obtenue sur la peau. Les résultats sont indiqués dans les tableaux 1 et 2 présentés ci-dessous :
Les caractéristiques des nacres utilisées dans ces exemples sont les suivantes :

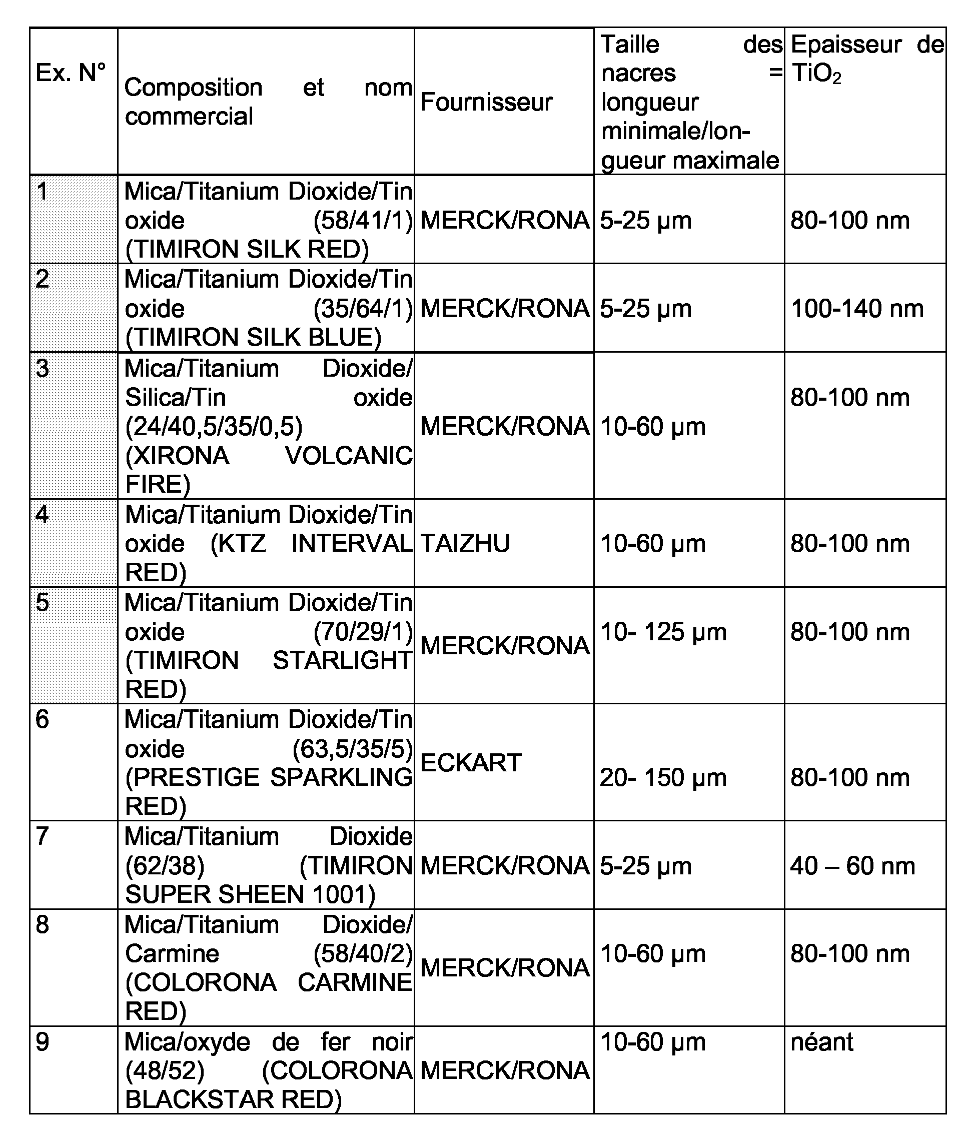

**Tableau 1 : exemples 1 à 5 selon l'invention**

| Composition | Ex.1 | Ex.2 | Ex.3 | Ex.4 | Ex. 5 |
|---|---|---|---|---|---|
| Phase A | | | | | |
| EUMULGIN BA 10® (1) | 5 | 5 | 5 | 5 | 5 |
| CERAPHYL 368® (2) | 66,2 | 66,2 | 66,2 | 66,2 | 66,2 |
| Propyl paraben | 0,15 | 0,15 | 0,15 | 0,15 | 0,15 |
| Methyl paraben | 0,15 | 0,15 | 0,15 | 0,15 | 0,15 |
| glycérine | 10 | 10 | 10 | 10 | 10 |
| Eau permutée | 13,75 | 13,75 | 13,75 | 13,75 | 13,75 |

| Phase B | | | | | |
|---|---|---|---|---|---|
| Aerosil 200® (3) | 1 | 1 | 1 | 1 | 1 |
| Ethanol | 2 | 2 | 2 | 2 | 2 |
| Chlorhexidine digluconate | 0,25 | 0,25 | 0,25 | 0,25 | 0,25 |
| Mica/Titanium Dioxide/Tin oxide (58/41/1) (TIMIRON SILK RED) | 1,5 | | | | |
| Mica/Titanium Dioxide/Tin oxide (35/64/1) (TIMIRON SILK BLUE) | | 1,5 | | | |
| Mica/Titanium Dioxide/ Silica/Tin oxide (24/40,5/35/0,5) (XIRONA VOLCANIC FIRE) | | | 1,5 | | |
| Mica/Titanium Dioxide/Tin oxide (KTZ INTERVAL RED) | | | | 1,5 | |
| Mica/Titanium Dioxide/Tin oxide (70/29/1) (TIMIRON STARLIGHT RED) | | | | | 1,5 |
| Total | 100 | 100 | 100 | 100 | 100 |
| Viscosité (Pa.s) | 2 | 2 | 2 | 2 | 2 |
| pH | 5,9 | 5,9 | 5,9 | 5,9 | 5,9 |
| Aspect dans le pot | Blanc nacré | Blanc nacré | Blanc nacré | Blanc nacré | Blanc nacré |
| Aspect lors de l'application sur carte de contraste | Couleur rose | Couleur bleue | Couleur rose | Couleur rose | Couleur rose |

| | | | | | |
|---|---|---|---|---|---|
| (1) Alcool béhénylique oxyéthyléné (10 OE) = EUMULGIN BA 10® commercialisé par la société Cognis (2) Ethylhexyl Palmitate = CERAPHYL 368® commercialisé par la société ISP (3) Silica = AEROSIL 200® commercialisé par la société Degussa-Hüls | | | | | |

**Tableau 2 : exemples comparatifs 6 à 9**

| Composition | Ex. 6 (comparatif) | Ex. 7 (comparatif) | Ex. 8 (comparatif) | Ex. 9 (comparatif) |
|---|---|---|---|---|
| EUMULGIN BA 10® (1) | 5 | 5 | 5 | 5 |
| CERAPHYL 368® (2) | 66,2 | 66,2 | 66,2 | 66,2 |
| Propyl paraben | 0,15 | 0,15 | 0,15 | 0,15 |
| Methyl paraben | 0,15 | 0,15 | 0,15 | 0,15 |
| glycérine | 10 | 10 | 10 | 10 |
| Eau permutée | 13,75 | 13,75 | 13,75 | 13,75 |
| Aerosil 200® (3) | 1 | 1 | 1 | 1 |
| Ethanol | 2 | 2 | 2 | 2 |
| Chlorhexidine digluconate | 0,25 | 0,25 | 0,25 | 0,25 |
| Mica/Titanium Dioxide/Tin oxide (63,5/35/5) (PRESTIGE SPARKLING RED) | 1,5 | | | |
| Mica/Titanium Dioxide (62/38) (TIMIRON SUPER SHEEN 1001) | | 1,5 | | |
| Mica/Titanium Dioxide/ Carmine (58/40/2) (COLORONA CARMINE RED) | | | 1,5 | |
| Mica/oxyde de fer noir (48/52) (COLORONA BLACKSTAR RED) | | | | 1,5 |
| Total | 100 | 100 | 100 | 100 |
| Viscosité (Pa.s) | 2 | 2 | 2 | 2 |
| pH | 5,9 | 5,9 | 5,9 | 5,9 |
| Aspect dans le pot | Blanc nacré | Blanc nacré | Rose | Brun foncé |
| Aspect lors de l'application sur carte de contraste | Faible effet rosé insuffisant | Pas d'effet de couleur | Pas de changement de couleur | Pas de changement de couleur |

| | | | | |
|---|---|---|---|---|
| (1) Alcool béhénylique oxyéthyléné (10 OE) = EUMULGIN BA 10® commercialisé par la société Cognis (2) Ethylhexyl Palmitate = CERAPHYL 368® commercialisé par la société ISP (3) Silica = AEROSIL 200® commercialisé par la société Degussa-Hüls | | | | |

Les exemples 1 à 5 montrent que l'effet de couleur est obtenu pour une épaisseur de TiO₂ supérieure à 80 nm (couleurs argent et or exclues) alors qu'avec une épaisseur inférieure à 80 nm (exemple comparatif 7), le résultat recherché ne peut pas être atteint.

Les exemples 1 à 5 montrent que l'effet de changement de couleur ne peut être obtenue que si la nacre a une longueur maximale inférieure à 130 µm alors qu'avec une longueur supérieure (exemple comparatif 6), l'effet ne peut pas être obtenu.

Les exemples comparatifs 8 et 9 montrent que l'effet de couleur ne peut pas être obtenu si les nacres sont colorées, et qu'il est donc indispensable d'utiliser des nacres non colorées.

### Exemple 10 selon l'invention : émulsion H/E

| Composition | Ex. 10 |
|---|---|
| Phase A | |
| CERAPHYL 368® (2) | 45,5 |
| DOW CORNING 245 FLUID® (4) | 14 |
| MARCOL 82® (5) | 10 |
| PARLEAM® (6) | 9 |

| Phase B | |
|---|---|
| Eau permutée | Qsp 100 |
| GLUCAMATE SSE 20 (7) | 4,6 |
| glycérine | 11 |
| conservateur | qs |
| Mica/Titanium Dioxide/Tin oxide (58/41/1) (TIMIRON SILK RED) | 1,5 |
| Viscosité (Pa.s) | 2 |
| pH | 5,9 |
| Aspect | Gel -crème souple blanche nacrée avec des reflets verts |
| Aspect lors de l'application sur la peau | Couleur verte |

| | |
|---|---|
| (2) Ethylhexyl palmitate = Ceraphyl 368® commercialisé par la société ISP (4) Cyclopentasiloxane = DOW CORNING 245 FLUID® commercialisé par la société DOW CORNING (5) Mineral oil = MARCOL 82® commercialisé par la société ESSO (6) Hydrogenated Polyisobutene = PARLEAM® commercialisé par la société NOF CORPORATION (7) PEG-20 methyl glucose sesquistearate = GLUCAMATE SSE 20® commercialisé par la société CHEMRON | |

Le mode opératoire est le suivant :
1- on mélange les constituants de la phase A,
2- on mélange les constituants de la phase B,
3- on chauffe les phases A et B à 80°C,
4- on introduit doucement sous agitation rapide, la phase aqueuse B dans la phase huileuse A.

On obtient une émulsion H/E.

### Exemple 11 selon l'invention : crème démaquillante (émulsion E/H)

| Composition | Ex. 11 |
|---|---|
| Phase A | |
| MARCOL 82® (5) | 3,2 |
| ISOPROPYL PALMITATE® (8) | 3 |
| DOW CORNING 5225C FORMULATION AID® (9) | 9,2 |

| Phase B | |
|---|---|
| Eau permutée | Qsp 100 |
| PEG-8 | 4 |
| Ethanol | 2,5 |
| Glycérine | 5 |
| Conservateur | 0,65 |
| Sel de Na d'EDTA | 0,1 |
| NaCl | 2,5 |
| TIMIRON STARLIGHT RED® | 3,5 |
| Aspect | Produit blanc |
| Aspect lors de l'application sur la peau | Produit blanc puis apparition de « perles » roses lors de la libération de l'eau sous forme de gouttelettes. |

| | |
|---|---|
| (5) Mineral oil = MARCOL 82® commercialisé par la société ESSO (8) Isopropyl Palmitate = Isopropyl Palmitate® commercialisé par la société Cognis (9) Cyclopentasiloxane (and) PEG/PPG-18/18 Dimethicone = DOW CORNING 5225C FORMULATION AID® commercialisé par la société Dow Corning. | |

Mode opératoire : On prépare la phase A par mélange des constituants sous agitation à 600 tours/minute. On prépare par ailleurs la phase B et on introduit une partie (environ 1/10) de la phase B dans la phase A très lentement sous agitation. On ajoute ensuite le reste de la phase B plus rapidement toujours sous agitation, et on maintient l'agitation pendant un certain temps.

On obtient une crème blanche ayant une viscosité mesurée au RHEOMAT 180, mobile 4, de 5,74 Pa.s (57,4 poises) au temps zéro. Cette viscosité se stabilise après 10 minutes à 4,56 Pa.s (45,6 poises).

### Exemple 12 selon l'invention : crème démaquillante E/H

| Composition | Ex.12 |
|---|---|
| Phase A | |
| L2724®(10) | 2,5 |
| Isohexadécane | 3,29 |
| PARLEAM® (6) | 2,47 |
| Cyclométhicone | 1,64 |
| Conservateur | 0,1 |

| Phase B | |
|---|---|
| Eau permutée | Qsp 100 |
| Sulfate de magnésium | 0,9 |
| Conservateur | 0,65 |
| TIMIRON SILK BLUE ® | 3 |
| Aspect | Produit blanc |
| Aspect lors de l'application sur la peau | Produit blanc puis apparition de « perles » roses lors de la libération de l'eau sous forme de gouttelettes. |

| | |
|---|---|
| (6) Hydrogenated Polyisobutene ; PARLEAM® commercialisé par la société NOF CORPORATION (10) polyoléfine à terminaison succinique ;L2724® commercialisé par la société LUBRIZOL | |

Mode opératoire : on introduit la phase aqueuse dans la phase huileuse comprenant les huiles et les émulsionnants, sous agitation, à une température allant de préférence d'environ 20 à 60°C.

### Exemple 13 selon l'invention : Emulsion H/E

| Composition | Ex.13 |
|---|---|
| Phase A | |
| EUMULGIN BA 10® (1) | 5 |
| CERAPHYL 368® (2) | 66,2 |
| Propyl paraben | 0,15 |
| Methyl paraben | 0,15 |
| glycérine | 10 |
| Eau permutée | 13,75 |

| Phase B | |
|---|---|
| Aerosil 200® (3) | 1 |
| Ethanol | 2 |
| Chlorhexidine digluconate | 0,25 |
| Mica/Titanium Dioxide/Tin oxide (58/41/1) (TIMIRON SILK RED) | 1,5 |
| 34 PC 3516 ULTRA BLUE BC® de Noveon (colorant) | 0,002 |
| Total | 100 |
| Viscosité (Pa.s) | 2 |
| pH | 5,9 |
| Aspect dans le pot | Bleue nacrée |
| Aspect lors de l'application sur la partie noire de la carte de contraste | Couleur violette |

| | |
|---|---|
| (1) Alcool béhénylique oxyéthyléné (10 OE) = EUMULGIN BA 10® commercialisé par la société Cognis (2) Ethylhexyl Palmitate = CERAPHYL 368® commercialisé par la société ISP (3) Silica = AEROSIL 200® commercialisé par la société Degussa-Hüls | |

## Revendications

1. Composition cosmétique à rincer, sous forme d'une émulsion contenant une phase huileuse et une phase aqueuse, **caractérisée en ce qu'**elle contient des particules interférentielles hydrophiles choisies parmi les nacres non colorées recouvertes d'une ou plusieurs couches d'un ou plusieurs oxydes métalliques, ces nacres ayant une longueur maximale inférieure ou égale à 130 µm, et l'épaisseur d'oxydes métalliques étant supérieure à 80 nm, la quantité de phase interne étant supérieure à 50 % en poids par rapport au poids total de la composition.

2. Composition selon la revendication 1, **caractérisée en ce qu'**en absence de colorant et/ou de pigment colorant, la composition a une clarté L* supérieure à 60 et une saturation C* inférieure à 10, mesurées dans l'espace colorimétrique CIE 1976.

3. Composition selon la revendication 1 ou 2, **caractérisée en ce que** les nacres ont une longueur allant de 5 à 130 µm.

4. Composition selon l'une quelconque des revendications précédentes, **caractérisée en ce que** les particules sont constituées de mica recouvert d'une couche d'oxyde de titane.

5. Composition selon la revendication précédente, **caractérisée en ce que** les particules interférentielles comprennent en outre une couche d'oxyde d'étain et/ou de dioxyde de silicium.

6. Composition selon l'une quelconque des revendications précédentes, **caractérisée en ce que** les particules interférentielles sont choisies parmi les nacres de type mica / oxyde de titane / oxyde d'étain, les nacres de type mica /oxyde de titane, et leurs mélanges.

7. Composition selon l'une quelconque des revendications précédentes, **caractérisée en ce que** les particules interférentielles sont présentes en une quantité allant de 0,5 à 5 % en poids par rapport au poids total de la composition.

8. Composition selon l'une quelconque des revendications précédentes, **caractérisée en ce que** la composition est une émulsion E/H ou H/E.

9. Composition selon l'une quelconque des revendications précédentes, **caractérisée en ce que** la quantité de phase interne va de 60 à 90% en poids par rapport au poids total de la composition.

10. Composition selon l'une quelconque des revendications précédentes, **caractérisée en ce que** la phase huileuse contient au moins une huile choisie parmi les huiles hydrocarbonées, les esters d'acide gras, les éthers d'alcool gras, et leurs mélanges.

11. Composition selon l'une quelconque des revendications précédentes, **caractérisée en ce qu'**elle comprend au moins un émulsionnant.

12. Composition selon l'une quelconque des revendications précédentes, **caractérisée en ce qu'**elle constitue une composition de nettoyage et.ou de démaquillage de la peau, des cheveux ou des muqueuses.

13. Utilisation cosmétique de la composition selon l'une quelconque des revendications 1 à 11, pour le démaquillage et/ou le nettoyage de la peau et/ou des cheveux et/ou des muqueuses, ou pour le soin de la peau.

14. Procédé de démaquillage et/ou de nettoyage de la peau et/ou des cheveux et/ou des muqueuses, ou de soin de la peau consistant :
1) à appliquer sur la zone à démaquiller ou à nettoyer ou à soigner, la composition selon l'une quelconque des revendications 1 à 11, en une épaisseur de 15 à 35 mg/cm²,
2) à masser la composition sur la zone pour l'étaler jusqu'à disparition de la couleur si la composition est une émulsion H/E ou jusqu'à apparition de la couleur si la composition est une émulsion E/H,
3) à rincer jusqu'à élimination de la composition.
